(19) Europäisches Patentamt
European Patent Office
Office européen des brevets

(11) Publication number : 0 352 609 B1

# (12) EUROPEAN PATENT SPECIFICATION

(45) Date of publication of patent specification : 20.01.93 Bulletin 93/03

(51) Int. Cl.$^5$ : A61K 31/70

(21) Application number : 89113194.8

(22) Date of filing : 19.07.89

## (54) Use of adenosine derivatives in preparing pharmaceutical compositions possessing immunostimulant activity.

(30) Priority : 29.07.88 IT 2157088

(43) Date of publication of application : 31.01.90 Bulletin 90/05

(45) Publication of the grant of the patent : 20.01.93 Bulletin 93/03

(84) Designated Contracting States : AT BE CH DE ES FR GB GR IT LI LU NL SE

(56) References cited :
FR-A- 2 313 937
FR-A- 2 354 100
GB-A- 2 074 446
JOURNAL OF SUPRAMOLECULAR STRUCTURE AND CELLULAR BIOCHEMISTRY, vol. 17, 1981, pages 11-25, Alan R. Liss, Inc.; J.I. TOOHEY:"Macrophages and methylthio groups in lymphocyte proliferation"
CANCER RESEARCH, vol. 46, October 1986, pages 5409-5412; J.H. FITCHEN et al.: "Methylthioadenosine phosphorylase deficiencyin human leukemias and solid tumors"
BIOCHEMICAL AND BIOPHYSICAL RESEARCH COMMUNICATIONS, vol. 70, no. 2, 1976, pages 622-629, Academic Press Inc.; C. BONA etal.: "Inhibition of mitogen induced blastogenesis by 5'-deoxy-5'-S-iso-butyl adenosine"
SCAND. J. IMMUNOL., vol. 20, 1984, pages 511-518; B.B. FREDHOLM et al.: "Effect of 5'-methylthioadenosine 3-deazaadenosine,and related compounds on human natural killer cell activity"
EXPERIMENTAL HEMATOLOGY, vol. 12, 1984, pages 867-871, International Society for Experimental Hematology; R.W. WOLFORD etal.: "Effect of 5'-methylthioadenosine (a naturally occurring nucleoside) on murine hematopoiesis"

(73) Proprietor : BIORESEARCH S.p.A.
Via Ciro Menotti 1/A
I-20129 milano (IT)

(72) Inventor : Gennari, Federico
Via Leonardo Da Vinci, 12
I-20060 Trucazzano (Milan) (IT)
Inventor : Bassi, Luigi
Via Torricelli, 10
I-26027 Rivolta d'Adda (Cremona) (IT)

(74) Representative : Gervasi, Gemma et al
NOTARBARTOLO & GERVASI Srl Viale Bianca Maria 33
I-20122 Milan (IT)

EP 0 352 609 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

(56) References cited :


**INT. J. IMMUNOPHARMAC., vol. 7, no. 2, 1985, pages 193-198, International Society for Immunopharmacology; F. DI PADOVA etal.: "Inhibition of lymphocyte function by a naturally occurring nucleoside: 5'-methylthioadenosine (MTA)"**

**CELL. IMMUNOL., vol. 49, no. 1, 1980, pages 26-33, Academic Press, Inc.; A.A. VANDENBARK et al.: "Inhibition of lymphocytetransformation by a naturally occurring metabolite: 5'-Methylthioadenosine"**

**Z. GESAMTE INN. MED., vol. 38, no. 3, 1983, pages 83-89; G. MÜLLER: "Immuninsuffizienz durch Enzymdefekte desPurinstoffwechsels"**

**J. LAB. CLIN. MED., vol. 100, no. 2, 1982, pages 269-278, The C.V. Mosby Co.; T.D. PALELLA et al.: "S-adenosylhomocysteineaccumulation and selective cytotoxicity in cultured T- and B-lymphoblasts"**

**PROC. NATL. ACAD. SCI. USA, vol. 77, no. 10, October 1980, pages 5639-5643; T.P. ZIMMERMAN et al.: "Modulation of cyclic AMPmetabolism by S-adenosylhomocysteine and S-3-deazaadenosylhomocysteine in mouse lymphocytes"**

**PROC. NATL. ACAD. SCI. USA, vol. 75, no. 8, August 1978, pages 3928-3932; M.C. PIKE et al.: "Requirement ofS-adenosyl-L-methionine-mediated methylation for human monocyte chemotaxis"**

**CLINICA CHEMICA ACTA, vol. 147, 1985, pages 15-23, Elsevier Science Publishers B.V. (Biomedical Division); G.C. MILLS et al.:"Urinary excretion of methylthioadenosine in immunodeficient children"**

**BIOCHEMICAL MEDICINE, no. 1, vol. 34, 1985, pages 37-51, Academic Press, Inc.; G.C. MILLS et al.: "Urinary excretion ofmodified purines and nucleosides in immunodeficient children"**

## Description

This invention relates to the use of adenosine derivatives for the preparation of pharmaceutical composition for the immunotheraphy of congenital and acquired deficiency and of tumours.

The therapeutic uses of the product class containing the products of the present invention as antivirals, cytostatics and oncogenic tissue transformation inhibitors are already known and widely described in the literature, for example in GB patent No. 1,555,991 in which the antitumoral activity of said compound is disclosed, but it is only directed to the prevention and treatment of a limited number of tumours that is viro-induced tumours.

The therapeutic uses of this product class as antiinflammatories, antipyretics, platelet antiaggregation agents and sleep inducers, claimed by the present Applicant in US patents 4,454,122 and 4,373,097, are also known.

J. Supramolecular Structure and cellular Biochemistry vol. 17 pages 11-17, 1981 discloses a generic study and hypothesis on the possible role of agents such as sulphydril compounds, disulfides, macrophages and methylthioadenosine in the proliferation of both normal and malignant cells. The information relative to methylthioadenosine which is the only compound named in this reference and related to the adenosine derivatives are only generic information about its hypothetic role as methylthiogroup donor in certain type cells.

In this reference there is no indication of any therapeutical use.

Reference should be made to the aforesaid patents for preparation details of the product of the present invention. The immunostimulants form a heterogeneous category of medicaments used for potentiating inadequate or depressed immune responses.

Of various nature and origin, they all have the capacity to activate both specific and natural immunity cell functions, either directly or via activation of endogenous mediators. In the great majority of cases they recognise more than one point of connection to the immune system, and are therefore able to stimulate various functions.

For many of them the real action mechanism is substantially unknowns, and this poses a limit to better understanding of their pharmacological potential and the interpretation of frequent side-effects which are often of considerable importance.

Immunostimulants are classified according to their origin, namely endogenous such as thymic hormones, monokines, lymphokines, interferons, or natural exogenous (generally obtained from microorganisms) such as BCG, Corynebacterium Parvum, Muramyl dipeptide, Bestatin and Lentinan, or also synthetis exogenous such as Levamisole, Isoprenosine and Tuftsin.

As the constantly increasing consumption figures prove, the interest in these medicaments is considerable but up to the present time more on a hypothetical use level rather than on the level of consistent results and real use.

The main fields of application of immunostimulants are the immunotherapy of congenital and acquired immunodeficiencies and tumour immunotherapy.

In the immunodeficiency field there is a current widespread use of thymic hormones, which restore normal resistance to infecting agents but raise considerable problems of selectivity, bioavailability and toxic effects which have not yet been overcome.

With regard to tumour therapy, since the immunogenicity of certain experimental tumours and the ability to induce their remission by acting on the immune functions of the host were demonstrated several years ago, numerous attempts have been directed towards establishing an immunotherapy for human tumours. These results have been discouraging, but in reality these were premature attempts the failure of which was caused by empiricism, poor knowledge of tumour biology and the interactions between the tumour and host, and the indiscriminate and improper use of immunostimulants.

Very intensive basic research is currently in progress directed towards an understanding of how tumours are generated and develop, the mechanisms by which they evade immunitary supervision, the reasons for their poor immunogenicity, and the importance of specific and natural immunity in protection against tumours.

This is a subject dense with unknowns, but undoubted successes obtained in some patients by immunotherapy formulas which included the use of LAK (lymphokine-activated killer cells), interleukin-2 and interferon have shown that immunotherapy can be effective (Rosemberg S.A. et al., A progress report on the treatment of 157 patients with advanced cancer using lymphokine-activated killer cells and interleukine-2 or high-dose interleukine-2 alone, N. Engl. J. Med. 316, 889, 1987; Goldstein D., Laszlo J., Interferon therapy in cancer: from imaginon to interferon, Cancer Res. 46, 4315, 1986).

In the present state of things it can be said that if immunotherapy is used rationally and is subjected to critical methodological selection, it opens extremely important perspectives, with considerable expectancy of the introduction of immunostimulants of known action mechanism, tested efficiency and low toxicity.

This invention relates to the use of adenosine derivatives as active principle in preparing pharmaceutical compositions possessing immunostimulant activity, for the immunotherapy of congenital and acquired immuno

deficiencies and of tumours comprising a pharmacologically acceptable carrier and an effective quantity of at least one of said adenosine derivatives of general formula:

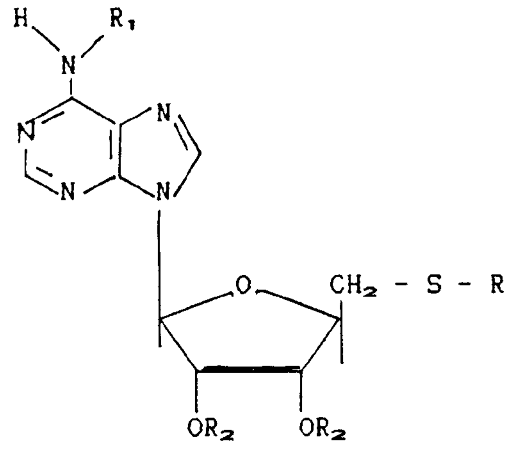

(I)

in which:

R = $C_1$-$C_{18}$ linear or branched alkyl radical, or phenylalkylene radical in which the alkylene chain has 1-6 C atoms

$R_1$ = H, $C_1$-$C_6$ aliphatic acyl radical, or $C_7$ aromatic acyl radical;

$R_2$ = H, $C_1$-$C_6$ aliphatic acyl radical, or $C_7$ aromatic acyl radical, or the two $R_2$ radicals together form an isopropylidene chain.

Moreover, when $R_1$ = H the invention also relates to pharmacologically acceptable acid addition salts of the compounds of formula (I).

Preferred meanings of R are: methyl, ethyl, propyl, isopropyl, butyl, isobutyl, sec-butyl, pentyl, hexyl, heptyl, octyl, decyl, hexadecyl, octadecyl, benzyl.

Preferred meanings of $R_1$ are: hydrogen, acetyl, propionyl, butyryl, succinyl, glutaryl, benzoyl, tosyl.

Preferred meanings of $R_2$ are: hydrogen, acetyl, propionyl, butyryl, succinyl, glutaryl, benzoyl, tosyl.

The preferred acid addition salts of the compounds of formula (I) are: chloride, sulphate, hydrogensulphate, phosphate, formate, acetate, citrate, tartrate, lactate, methanesulphonate, p-toluenesulphonate.

According to the present invention, we have now discovered that the adenosine derivatives of formula (I) possess considerable immunostimulant activity in addition to their already known pharmacological activities.

The immunostimulant activity was initially demonstrated for certain of the terms of general formula (I) by using four experimental tests, the results of which are given in Tables A, B, C and D.

The following experimental tests were used:

1. Primary antibody response against thymus-dependent antigen, evaluated as the number of plaque-forming splenic cells (PFC) Mice B6 D2 F1, antigen SRBC (red sheep corpuscles)

Jerne N.K and Nordin A.A., Plaque formation in ager by single antibody-producing cells Science, 140, 405, 1963.

Table A

2. Delayed hypersensitivity against cell antigen (DTH) Mice B6 D2 F1, antigen SRBC (red sheep corpuscles)

Lagrange P.H., Mackaness G.B and Miller T.E., potentiation of T-cell-mediated immunity by selective suppression of antibody formation with cyclophosphamide. J. Exp. Med., 139, 1529, 1974.

Table B

3. Macrophage cytotoxic activity against target tumour induced by Corynebacterium Parvum

Mice C57 BL/6; tumour L1210 pretagged with $^{51}Cr$; attack:target ratio 80:1; tumouricide activity evaluated as release of tagging compound and expressed as % of specific cytotoxicity. Herscowitr H.B. et al., Eds., Manual of macrophage methodology, M. Dekker 1981.

Table C

4. Splenic NK (natural cytotoxic cell) activity potentiated with polynucleotide (Poly I:C)

Mice C57 BL/6; tumour YAC-1; evaluation as in test 3. Herbeman R.B. Ed., "Natural cell-mediated immunity against tumors". Academic Press, 1980.

Table D

TABLE A

Effect of compounds on antibody response (P.F.C.)

| Compound of formula (I) | Orally administered dose (mg/kg) | % increase in PFC/spleen vs. control |
|---|---|---|
| R=CH3, R1=R2=H (MTA) | 5 | 80 |
| R=CH3, R1=R2=H (MTA) | 20 | 105 |
| R=CH3, R1=R2=H (MTA) | 40 | 115 |
| R=CH3, R1=R2=H (MTA) | 100 | 40 |
| R=CH2-CH3, R1=R2=H | 5 | 70 |
| R=CH2-CH3, R1=R2=H | 20 | 100 |
| R=CH2-CH3, R1=R2=H | 40 | 110 |
| R=CH2-CH3, R1=R2=H | 100 | 50 |
| R=isobutyl, R1=R2=H | 5 | 75 |
| R=isobutyl, R1=R2=H | 20 | 95 |
| R=isobutyl, R1=R2=H | 40 | 105 |
| R=isobutyl, R1=R2=H | 100 | 45 |
| R=(CH2)2-CH3, R1=R2=H | 20 | 95 |
| R=(CH2)2-CH3, R1=R2=H | 40 | 100 |
| R=(CH2)4-CH3, R1=R2=H | 20 | 90 |
| R=(CH2)4-CH3, R1=R2=H | 40 | 95 |
| R=isopropyl, R1=R2=H | 40 | 90 |
| R=(CH2)3-CH3, R1=R2=H | 40 | 100 |
| R=sec-butyl, R1=R2=H | 40 | 95 |
| R=(CH2)5-CH3, R1=R2=H | 40 | 90 |
| R=(CH2)6-CH3, R1=R2=H | 40 | 90 |
| R=(CH2)7-CH3, R1=R2=H | 40 | 85 |
| R=(CH2)9-CH3, R1=R2=H | 40 | 85 |
| R=(CH2)11-CH3, R1=R2=H | 40 | 80 |
| R=(CH2)15-CH3, R1=R2=H | 40 | 70 |
| R=(CH2)17-CH3, R1=R2=H | 40 | 60 |
| R=benzyl, R1=R2=H | 40 | 90 |

| | | |
|---|---|---|
| R=CH3, R1=H, R2=acetyl | 40 | 95 |
| R=CH3, R1=H, R2=butyryl | 40 | 90 |
| R=CH3, R1=H, R2=succinyl | 40 | 85 |
| R=CH3, R1=H, R2=benzoyl | 40 | 75 |
| R=CH3, R1=H, R2=tosyl | 40 | 60 |
| R=CH2-CH3, R1=H, R2=acetyl | 40 | 90 |
| R=CH2-CH3, R1=H, R2=benzoyl | 40 | 60 |
| R=isobutyl, R1=H, R2=acetyl | 40 | 95 |
| R=isobutyl, R1=H, R2=butyryl | 40 | 90 |
| R=CH3, R1=R2=acetyl | 40 | 70 |
| R=CH3, R1=R2=benzoyl | 40 | 40 |
| R=CH2-CH3, R1=R2=acetyl | 40 | 70 |
| R=isobutyl, R1=R2=acetyl | 40 | 75 |
| R=CH3, R1=H, R2-R2=isopropylidene | 40 | 95 |
| Tuftsin 25 gamma i.p. 6 days before immunization | | 65 |

All the examined products are able to potentiate the primary antibody response to thymus-dependent antigen, as shown by the percentage increases in the splenic PFC number given in Table A. The most effective compound of the series appears to be MTA which at doses of 20 and 40 mg/kg doubles the number of plaque forming cells.

The treatment was carried out during the inductive stage of the response.

The effect of a known immunostimulant, Tuftsin, is shown at the foot of Table A.

Similar potentiation is observed in a cell immunity model for which daily treatment during the sensitization period results in amplified DTH response, as shown by Table B.

TABLE B

Effect of compounds on delayed hypersensitivity (DTH)

| Compound of formula (I) | Orally administered dose (mg/kg) | % increase in paw thickening vs. control |
|---|---|---|
| R=CH3, R1=R2=H (MTA) | 10 | 105 |
| R=CH3, R1=R2=H (MTA) | 50 | 65 |
| R=CH3, R1=R2=H (MTA) | 300 | 60 |
| R=CH2-CH3, R1=R2=H | 10 | 100 |
| R=CH2-CH3, R1=R2=H | 50 | 65 |
| R=CH2-CH3, R1=R2=H | 300 | 50 |
| R=isobutyl, R1=R2=H | 10 | 95 |
| R=isobutyl, R1=R2=H | 50 | 60 |
| R=isobutyl, R1=R2=H | 300 | 50 |
| R=(CH2)2-CH3, R1=R2=H | 10 | 90 |
| R=(CH2)4-CH3, R1=R2=H | 10 | 85 |
| R=isopropyl, R1=R2=H | 10 | 95 |
| R=(CH2)3-CH3, R1=R2=H | 10 | 90 |
| R=sec-butyl, R1=R2=H | 10 | 85 |
| R=(CH2)5-CH3, R1=R2=H | 10 | 80 |
| R=(CH2)6-CH3, R1=R2=H | 10 | 80 |
| R=(CH2)7-CH3, R1=R2=H | 10 | 70 |
| R=(CH2)9-CH3, R1=R2=H | 10 | 65 |
| R=(CH2)11-CH3, R1=R2=H | 10 | 55 |
| R=(CH2)15-CH3, R1=R2=H | 10 | 40 |
| R=(CH2)17-CH3, R1=R2=H | 10 | 30 |
| R=benzyl, R1=R2=H | 10 | 45 |
| R=CH3, R1=H, R2=acetyl | 10 | 95 |
| R=CH3, R1=H, R2=butyryl | 10 | 90 |
| R=CH3, R1=H, R2=succinyl | 10 | 70 |
| R=CH3, R1=H, R2=benzoyl | 10 | 50 |
| R=CH3, R1=H, R2=tosyl | 10 | 40 |
| R=CH2-CH3, R1=H, R2=acetyl | 10 | 80 |
| R=CH2-CH3, R1=H, R2=benzoyl | 10 | 45 |
| R=isobutyl, R1=H, R2=acetyl | 10 | 90 |
| R=isobutyl, R1=H, R2=butyryl | 10 | 80 |
| R=CH3, R1=R2=acetyl | 10 | 75 |

| | | |
|---|---|---|
| R=CH3, R1=R2=benzoyl | 10 | 40 |
| R=CH2-CH3, R1=R2=acetyl | 10 | 70 |
| R=isobutyl, R1=R2=acetyl | 10 | 70 |
| R=CH3, R1=H, R2-R2=isopropylidene | 10 | 80 |
| Muramyl dipeptide 1 gamma i.p. on day of immunization | | 50 |

In this case MTA is again seen to be the most active compound of the series under examination. with maximum efficiency for a dose of 10 mg/kg.

The effect of another known immunostimulant, Muramyl dipeptide (MDP), is shown at the foot of the table.

For certain of the products under examination immunostimulant capacity was also evaluated on a model of macrophage cytotoxic activity against target tumour induced by Corynebacterium Parvum.

Table C shows that all the tested compounds are able to potentiate the inducing effect of Corynebacterium Parvum.

By associating adenosine derivatives of general formula (I) with a sub-optimum dose of Corynebacterium, the obtained microphage activation is equal to or very often greater than that obtained by an optimum dose of Corynebacterium.

Compounds administered in the absence of activator are unable to stimulate the microphage cytotoxic function.

TABLE C

Effect of compounds on cytotoxic activity of macrophages activated by Corynebacterium Parvum

| Compound of formula (I) | Orally administered dose (mg/kg) | % specific cytotoxicity spontaneous | CB200 mouse | CB700 mouse |
|---|---|---|---|---|
| --- | --- | 2.5 | 15.4 | 34.3 |
| R=CH3, R1=R2=H (MTA) | 5 | 4.6 | 42.1 | 34.6 |

| | | | | |
|---|---|---|---|---|
| R=CH3, R1=R2=H (MTA) | 25 | 3.5 | 40.3 | 33.8 |
| R=CH2-CH3, R1=R2=H | 5 | 2 | 38.2 | 34.1 |
| R=CH2-CH3, R1=R2=H | 25 | 3 | 36.1 | 33.7 |
| R=isobutyl, R1=R2=H | 5 | 4 | 39.3 | 33.9 |
| R=isobutyl, R1=R2=H | 25 | 4.1 | 35.2 | 33.5 |
| R=(CH2)2-CH3, R1=R2=H | 5 | 2.3 | 36.3 | 33.7 |
| R=(CH2)4-CH3, R1=R2=H | 5 | 2.8 | 35.4 | 34.6 |
| R=isopropyl, R1=R2=H | 5 | 3 | 38.3 | 33.9 |
| R=(CH2)3-CH3, R1=R2=H | 5 | 2.6 | 39.4 | 33.7 |
| R=sec-butyl, R1=R2=H | 5 | 2.5 | 40.1 | 34.5 |
| R=(CH2)5-CH3, R1=R2=H | 5 | 2.4 | 36.3 | 33.8 |
| R=(CH2)6-CH3, R1=R2=H | 5 | 3 | 33.2 | 34.1 |
| R=(CH2)7-CH3, R1=R2=H | 5 | 4 | 30.7 | 33.9 |
| R=(CH2)9-CH3, R1=R2=H | 5 | 3 | 28.6 | 34.2 |
| R=benzyl, R1=R2=H | 5 | 2.6 | 27.4 | 33.6 |
| R=CH3, R1=H, R2=acetyl | 5 | 3 | 33.6 | 33.1 |
| R=CH3, R1=H, R2=butyryl | 5 | 2 | 31.7 | 34.3 |
| R=isobutyl, R1=H, R2=acetyl | 5 | 1.8 | 32.6 | 34.5 |
| R=isobutyl, R1=H, R2=butyryl | 5 | 1.6 | 31.5 | 33.9 |
| R=CH3, R1=R2=acetyl | 5 | 2 | 29.7 | 34.1 |
| R=isobutyl, R1=R2=acetyl | 5 | 5 | 29.8 | 34.3 |
| R=CH3, R1=H, R2-R2=isopropylidene | 5 | 3.4 | 31.6 | 33.7 |

Of all the prepared products, that which proved of particular interest from the point of view of the present invention was 5′-deoxy-5′-methylthioadenosine (MTA) of formula (II):

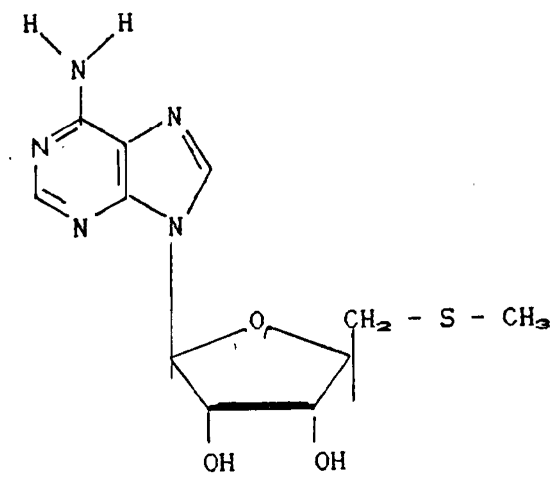

(II)

which is a physiological compound already present in living organisms and proved to be the most active in every test carried out.

A further evaluation of the stimulant capacity of MTA was made on an NK activity model.

Table D shows that when administered alone to the animal, the product does not modify the spontaneous NK activity. However, when associated with Poly I:C it potentiates its effect by reducing the polynucleotide dose required to obtain maximum activation.

TABLE D

Effect of MTA on splenic NK activity potentiated by Poly I:C

| | Orally administered dose (mg/kg) | % specific cytotoxicity spontaneous | Poly I:C 25/mouse | Poly I:C 25/mouse |
|---|---|---|---|---|
| | --- | 14 | 31 | 52 |
| MTA | 15 | 17 | 53 | 56 |

In general, the tested products influence specific immunity by amplifying both humoral and cell-mediated immune responses. They do not appear to directly influence natural immunity, but when associated with conventional macrophage and NK function inducers they considerably potentiate their effect, so greatly reducing the activator dose required for optimum response.

The compounds of the present invention are practically free of acute toxicity when administered orally. They are also practically free of toxicity at therapeutic doses for any method of administration.

The following values apply to MTA:

| | |
|---|---|
| $LD_{50}$ in the mouse for oral administration | 2000 mg/kg |
| " " i.v. " | 360 mg/kg |

The adenosine derivatives of formula (I) can be administered orally, parenterally, intravenously or rectally in any therapeutically useful form, either alone or diluted with suitable pharmacologically acceptable excipients. They can also be used in products for external topical application, and in other forms normally used in the pharmaceutical field.

The active principle content of the pharmaceutical compositions of the present invention corresponds to a therapeutic administration of between 5 and 20 mg/kg of body weight.

Some examples of typical pharmaceutical compositions comprising MTA are given hereinafter by way of illustration:

100 mg capsules

| | |
|---|---|
| MTA | 100 mg |
| Mannitol | 195 mg |
| Magnesium stearate | 5 mg |
| | 300 mg |

50 mg capsules

| | |
|---|---|
| MTA | 50 mg |
| Mannitol | 100 mg |
| Magnesium stearate | 3 mg |
| | 153 mg |

100 mg tablets

| | |
|---|---|
| MTA | 100 mg |
| Starch | 100 mg |
| Magnesium stearate | 15 mg |
| Lactulose | 85 mg |
| | 300 mg |

50 mg tablets

| | |
|---|---|
| MTA | 50 mg |
| Starch | 120 mg |
| Magnesium stearate | 15 mg |
| Lactulose | 115 mg |
| | 300 mg |

100 mg suppositories

| | |
|---|---|
| MTA | 100 mg |
| Suppository mass | 1700 mg |
| | 1800 mg |

50 mg suppositories

| | |
|---|---|
| MTA | 50 mg |
| Suppository mass | 1450 mg |
| | 1500 mg |

50 mg injectable vial

| | |
|---|---|
| MTA.HCl 56.15 mg, equivalent to | |
| MTA | 50 mg |
| Lidocaine HCl | 25 mg |
| Water to make up to | 3 ml |

25 mg injectable vial

| | |
|---|---|
| MTA.HCl 56.15 mg, equivalent to | |
| MTA | 25 mg |
| Lidocaine HCl | 20 mg |
| Water to make up to | 2 ml |

100 mg oral vial

| | |
|---|---|
| MTA.HCl 112.3 mg, equivalent to | |
| MTA | 100 mg |
| Citrus flavouring | 0.025 mg |
| Sugar | 1 g |
| Antifermenting agent | 50 mg |
| Water to make up to | 5 ml |

50 mg oral vial

| | |
|---|---|
| MTA.HCl 56.15 mg, equivalent to | |
| MTA | 50 mg |
| Citrus flavouring | 0.015 mg |
| Sugar | 0.5 g |
| Antifermenting agent | 30 mg |
| Water to make up to | 5 ml |

## Claims

**Claims for the following Contracting States : AT, BE, CH, DE, FR, GB, IT, LI, LU, NL, SE**

**1.** The use of adenosine derivatives of general formula (I):

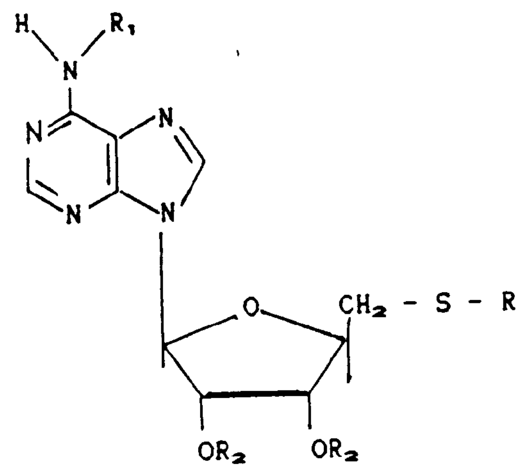

(I)

in which:

R = $C_1$-$C_{18}$ linear or branched alkyl radical, or phenylalkylene radical in which the alkylene chain has 1-6 C atoms;

$R_1$ = H, $C_1$-$C_6$ aliphatic acyl radical, or $C_7$ aromatic acyl radical;

$R_2$ = H, $C_1$-$C_6$ aliphatic acyl radical, or $C_7$ aromatic acyl radical, or the two $R_2$ radicals together form an isopropylidene chain;

as active principle for the preparation of pharmaceutical compositions for the immunotherapy of congenital and acquired immunodeficiencies and of tumours.

2. The use of adenosine derivatives as claimed in claim 1, characterised in that R is a group chosen from methyl, ethyl, propyl, isopropyl, butyl, isobutyl, sec-butyl, pentyl, hexyl, heptyl, octyl, decyl, hexadecyl, octadecyl, benzyl; $R_1$ is a group chosen from hydrogen, acetyl, propionyl, butyryl, succinyl, glutaryl, benzoyl, tosyl; and $R_2$ is a group chosen from hydrogen, acetyl, propionyl, butyryl, succinyl, glutaryl, benzoyl, tosyl.

3. The use of adenosine derivatives as claimed in claim 1, characterised in that $R_1$=$R_2$=H, R=$CH_3$.

4. The use of adenosine derivatives as claimed in claim 1, characterised by including the use of a pharmacologically acceptable acid addition salt when $R_1$ = H.

5. The use of adenosine derivatives as claimed in claim 4, characterised in that the acid addition salt is chosen from chloride, sulphate, hydrogensulphate, phosphate, formate, acetate, citrate, tartrate, lactate, methanesulphonate, p-toluensulphonate.

6. The use of adenosine derivatives as claimed in claim 1, characterised by the fact that said compositions are suitable for administration by oral, parenteral, intravenous, rectal or external topical route.

7. The use of adenosine derivatives as claimed in claim 1, characterised by the fact that said compositions are prepared in the form of capsules containing from 50 to 100 mg of derivative of formula (I) where $R_1$=$R_2$=H, R=$CH_3$.

8. The use of adenosine derivatives as claimed in claim 1, characterised by the fact that said compositions are prepared in the form of tablets containing from 50 to 100 mg of derivative of formula (I) where $R_1$=$R_2$=H, R=$CH_3$.

9. The use of adenosine derivatives as claimed in claim 1, characterised by the fact that said compositions are prepared in the form of suppositories containing from 50 to 100 mg of derivative of formula (I) where $R_1$=$R_2$=H, R=$CH_3$.

10. The use of adenosine derivatives as claimed in claim 1, characterised by the fact that said compositions are prepared in the form of injectable vials containing from 25 to 50 mg of derivative of formula (I) where $R_1$=$R_2$=H, R=$CH_3$.

11. The use of adenosine derivatives as claimed in claim 1, characterised by the fact that said compositions are prepared in the form of vials for oral use containing from 50 to 100 mg of derivative of formula (I) where $R_1$=$R_2$=H, R=$CH_3$.

**Claims for the following Contracting States : GR, ES**

1. A method for preparing pharmaceutical compositions for the immunotherapy of congenital and acquired immunodeficiencies and of tumors containing as active principle an adenosine derivative of general formula (I):

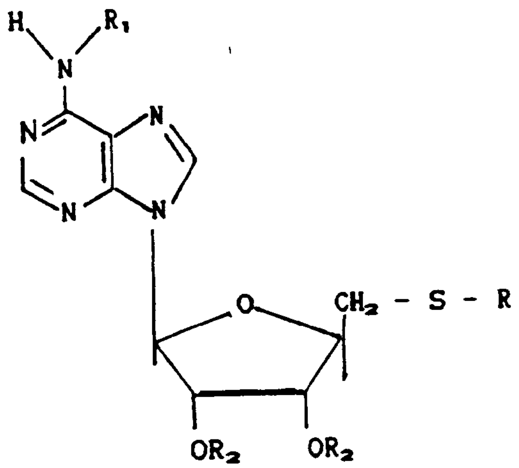

(I)

in which:
R=$C_1$-$C_{18}$ linear or branched alkyl radical, or phenylalkylene radical in which the alkylene chain has 1-6 C atoms;
$R_1$ = H, $C_1$-$C_6$ aliphatic acyl radical, or $C_7$ aromatic acyl radical;
$R_2$ = H, $C_1$-$C_6$ aliphatic acyl radical, r $C_7$ aromatic acyl radical, or the two $R_2$ radicals together form an isopropylidene chain, in combination with suitable pharmaceutically acceptable excipients, comprising the admixture of said adenosine derivative with said excipients.

2. A method as claimed in claim 1, characterised in that R is a group chosen from metyl, ethyl, propyl, isopropyl, butyl, isobutyl, sec-butyl, pentyl, hexyl, heptyl, octyl, decyl, hexadecyl, octadecyl, benzyl; $R_1$ is a group chosen from hydrogen, acetyl, propionyl, butyryl, succinyl, glutaryl, benzoyl, tosyl; and $R_2$ is a group chosen from hydrogen, acetyl, propionyl, butyryl, succinyl, glutaryl, benzoyl, tosyl.

3. A method as claimed in claim 1, characterised in that $R_1$=$R_2$=H, R=$CH_3$.

4. A method as claimed in claim 1, characterised by including the use of a pharmacologically acceptable acid addition salt when $R_1$= H.

5. A method as claimed in claim 4, characterised in that the acid addition salt is chosen from chloride, sulphate, hydrogensulphate, phosphate, formate, acetate, citrate, tartrate, lactate, methanesulphonate, p-toluensulphonate.

6. A method as claimed in claim 1, characterised by the fact that said compositions are suitable for administration by oral, parenteral, intravenous, rectal or external topical route.

7. A method as claimed in claim 1, charactetrised by the fact that said compositions are prepared in the form of capsules containing from 50 to 100 mg of derivative of formula (I) where $R_1$=$R_2$=H, R=$CH_3$.

8. A method as claimed in claim 1, characterised by the fact that said compositions are prepared in the form of tablets containing from 50 to 10 mg of derivative of formula (I) where $R_1R_2$=H, R=$CH_3$.

9. A method as claimed in claim 1, characterised by the fact that said compositions are prepared in the form of suppositories containing from 50 to 100 mg of derivative of formula (I) where $R_1$=$R_2$=H, R=$CH_3$.

10. A method as claimed in claim 1, characterised by the fact that said compositions are prepared in the form of injectable vials containing from 25 to 50 mg of derivatives of formula (I) where $R_1$=$R_2$=H, R=$CH_3$.

11. A method as claimed in claim 1, characterised by the fact that said compositions are prepared in the form of vials for oral use containing from 50 to 100 mg of derivative of formula (I) where $R_1$=$R_2$=H, R=$CH_3$.

**Patentansprüche**

**Patentansprüche für folgende Vertragsstaaten : AT, BE, CH, DE, FR, GB, IT, LI, LU, NL, SE**

1. Verwendung von Adenosinderivaten der allgemeinen Formel (I)

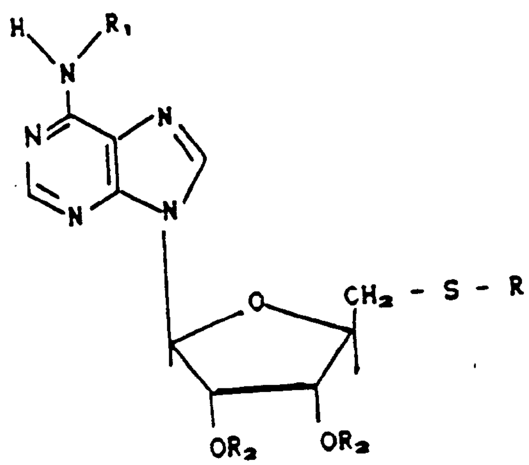

(I)

worin:
R = ein geradekettiger oder verzweigter $C_1$-$C_{18}$-Alkylrest oder ein Phenylalkylenrest, dessen Alkylenkette 1-6 C-Atome aufweist;
$R_1$ = H, ein $C_1$-$C_6$-aliphatischer Acylrest oder ein $C_7$-aromatischer Acylrest;
$R_2$ = H, ein $C_1$-$C_6$-aliphatischer Acylrest oder ein $C_7$-aromatischer Acylrest oder die zwei Reste $R_2$ bilden gemeinsam eine Isopropylidenkette;
als Wirkstoff zur Herstellung von Arzneimittelzubereitungen für die Immuntherapie von angeborener und erworbener Immundefizienz und von Tumoren.

2. Verwendung von Adenosinderivaten gemäß Anspruch 1, dadurch gekennzeichnet, daß R eine unter Methyl, Ethyl, Propyl, Isopropyl, Butyl, Isobutyl, sec-Butyl, Pentyl, Hexyl, Heptyl, Octyl, Decyl, Hexadecyl, Octadecyl, Benzyl ausgewählte Gruppe, $R_1$ eine unter Wasserstoff, Acetyl, Propionyl, Butyryl, Succinyl, Glutaryl, Benzoyl, Tosyl ausgewählte Gruppe und $R_2$ eine unter Wasserstoff, Acetyl, Propionyl, Butyryl, Succinyl, Glutaryl, Benzoyl, Tosyl ausgewählte Gruppe bedeuten.

3. Verwendung von Adenosinderivaten gemäß Anspruch 1, dadurch gekennzeichnet, daß $R_1$ = $R_2$ = H, R = $CH_3$.

4. Verwendung von Adenosinderivaten gemäß Anspruch 1, dadurch gekennzeichnet, daß die Verwendung eines pharmakologisch geeigneten Säure-Additionssalzes eingeschlossen ist, wenn $R_1$ = H.

5. Verwendung von Adenosinderivaten gemäß Anspruch 4, dadurch gekennzeichnet, daß das Säure-Additionssalz unter Chlorid, Sulfat, Hydrogensulfat, Phosphat, Formiat, Acetat, Citrat, Tartrat, Lactat, Methansulfonat und p-Toluolsulfonat ausgewählt ist.

6. Verwendung von Adenosinderivaten gemäß Anspruch 1, dadurch gekennzeichnet, daß die Zubereitungen geeignet sind für die Verabreichung auf oralem, parenteralem, intravenösem, rektalem oder äußerlich topischem Weg.

7. Verwendung von Adenosinderivaten gemäß Anspruch 1, dadurch gekennzeichnet, daß die Zubereitungen

in Form von Kapseln ausgebildet sind, die 50 bis 100 mg des Derivats der Formel (I) enthalten, worin $R_1$ = $R_2$ = H, R = $CH_3$.

8. Verwendung von Adenosinderivaten gemäß Anspruch 1, dadurch gekennzeichnet, daß die Zubereitungen in Form von Tabletten ausgebildet sind, die 50 bis 100 mg des Derivats der Formel (I) enthalten, worin $R_1$ = $R_2$ = H, R = $CH_3$.

9. Verwendung von Adenosinderivaten gemäß Anspruch 1, dadurch gekennzeichnet, daß die Zubereitungen in Form von Suppositorien ausgebildet sind, die 50 bis 100 mg des Derivats der Formel (I) enthalten, worin $R_1$ = $R_2$ = H, R = $CH_3$.

10. Verwendung von Adenosinderivaten gemäß Anspruch 1, dadurch gekennzeichnet, daß die Zubereitungen in Form von Fläschchen mit Injektionsflüssigkeit ausgebildet sind, die 25 bis 50 mg des Derivats der Formel (I) enthalten, worin $R_1$ = $R_2$ = H, R = $CH_3$.

11. Verwendung von Adenosinderivaten gemäß Anspruch 1, dadurch gekennzeichnet, daß die Zubereitungen in Form von Fläschchen mit der Zusammensetzung für orale Verabreichung ausgebildet sind, die 50 bis 100 mg des Derivats der Formel (I) enthalten, worin $R_1$ = $R_2$ = H, R = $CH_3$.

**Patentansprüche für folgende Vertragsstaaten : GR, ES**

1. Verfahren zur Herstellung von pharmazeutischen Zubereitungen für die Immuntherapie von angeborener und erworbener Immundefizienz und von Tumoren, welche als Wirkstoff ein Adenosinderivat der allgemeinen Formel (I)

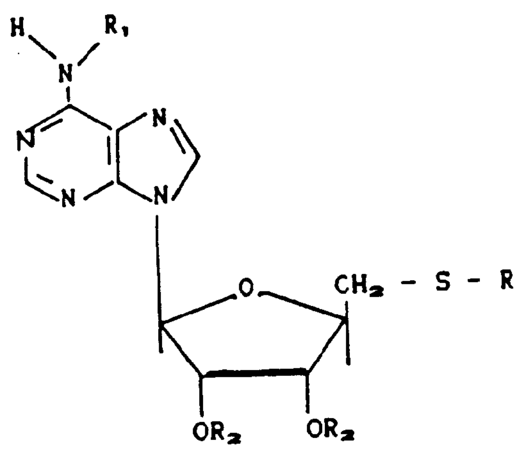

(I)

worin:
R = ein geradekettiger oder verzweigter $C_1$-$C_{18}$-Alkylrest oder ein Phenylalkylenrest, dessen Alkylenkette 1-6 C-Atome aufweist;
$R_1$ = H, ein $C_1$-$C_6$-aliphatischer Acylrest oder ein $C_7$-aromatischer Acylrest;
$R_2$ = H, ein $C_1$-$C_6$-aliphatischer Acylrest oder ein $C_7$-aromatischer Acylrest oder die zwei Reste $R_2$ bilden gemeinsam eine Isopropylidenkette;
in Kombination mit passenden pharmazeutisch geeigneten Exzipienten enthalten, welches das Vermischen dieses Adenosinderivats mit den Exzipienten umfaßt.

2. Verfahren gemäß Anspruch 1, dadurch gekennzeichnet, daß R eine unter Methyl, Ethyl, Propyl, Isopropyl, Butyl, Isobutyl, sec-Butyl, Pentyl, Hexyl, Heptyl, Octyl, Decyl, Hexadecyl, Octadecyl, Benzyl ausgewählte Gruppe, $R_1$ eine unter Wasserstoff, Acetyl, Propionyl, Butyryl, Succinyl, Glutaryl, Benzoyl, Tosyl ausgewählte Gruppe und $R_2$ eine unter Wasserstoff, Acetyl, Propionyl, Butyryl, Succinyl, Glutaryl, Benzoyl, Tosyl ausgewählte Gruppe bedeuten.

3. Verfahren gemäß Anspruch 1, dadurch gekennzeichnet, daß $R_1$ = $R_2$ = H, R = $CH_3$.

4. Verfahren gemäß Anspruch 1, dadurch gekennzeichnet, daß es die Verwendung eines pharmakologisch

geeigneten Säure-Additionssalzes umfaßt, wenn $R_1$ = H.

5. Verfahren gemäß Anspruch 4, dadurch gekennzeichnet, daß das Säure-Additionssalz unter Chlorid, Sulfat, Hydrogensulfat, Phosphat, Formiat, Acetat, Citrat, Tartrat, Lactat, Methansulfonat und p-Toluolsulfonat ausgewählt ist.

6. Verfahren gemäß Anspruch 1, dadurch gekennzeichnet, daß die Zubereitungen geeignet sind für die Verabreichung auf oralem, parenteralem, intravenösem, rektalem oder äußerlich topischem Weg.

7. Verfahren gemäß Anspruch 1, dadurch gekennzeichnet, daß die Zubereitungen in Form von Kapseln ausgebildet sind, die 50 bis 100 mg dees Derivats der Formel (I) enthalten, worin $R_1 = R_2$ = H, R = $CH_3$.

8. Verfahren gemäß Anspruch 1, dadurch gekennzeichnet, daß die Zubereïtungen in Form von Tabletten ausgebildet sind, die 50 bis 100 mg des Derivats der Formel (I) enthalten. worin $R_1 = R_2$ = H, R = $CH_3$.

9. Verfahren gemäß Anspruch 1, dadurch gekennzeichnet, daß die Zubereitungen in Form von Suppositorien ausgebildet sind, die 50 bis 100 mg des Derivats der Formel (I) enthalten, worin $R_1 = R_2$ = H, R = $CH_3$.

10. Verfahren gemäß Anspruch 1, dadurch gekennzeichnet, daß die Zubereitungen in Form von Fläschchen mit Injektionsflüssigkeit ausgebildet sind, die 25 bis 50 mg des Derivats der Formel (I) enthalten, worin $R_1 = R_2$ = H, R = $CH_3$.

11. Verfahren gemäß Anspruch 1, dadurch gekennzeichnet, daß die Zubereitungen in Form von Fläschchen mit der Zusammensetzung für orale Verabreichung ausgebildet sind, die 50 bis 100 mg des Derivats der Formel (I) enthalten, worin $R_1 = R_2$ = H, R = $CH_3$.

## Revendications

### Revendications pour les Etats contractants suivants : AT, BE, CH, DE, FR, GB, IT, LI, LU, NL, SE

1. Utilisation de dérivés de l'adénosine de formule générale (I) :

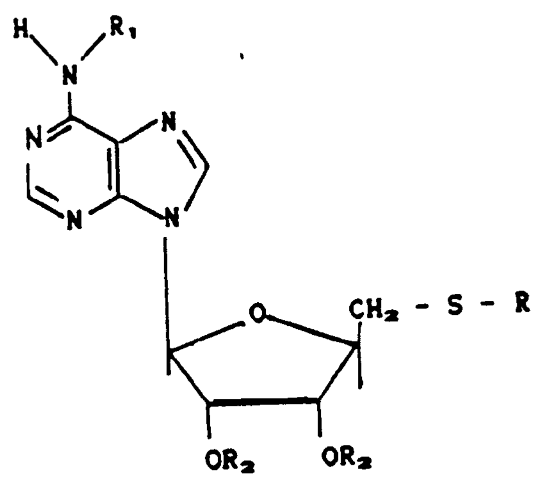

(I)

dans laquelle :

R = $C_1$-$C_{18}$ est un radical alkyle linéaire ou ramifié ou un radical phénylalkylène dans lequel la chaîne alkylène a de 1 à 6 atomes de carbone;

$R_1$ = H, $C_1$-$C_6$ est un radical acyle aliphatique ou un radical acyle aromatique $C_7$;

$R_2$ = H, $C_1$-$C_6$ est un radical acyle aliphatique, ou $C_7$ un radical acyle aromatique, ou les deux radicaux $R_2$ forment ensemble une chaîne isopropylidène;

comme principe actif pour la préparation de composés pharmaceutiques immunostimulants pour le traitement thérapeutique d'immunodéficiences congénitales ou acquises et de tumeurs.

2. Utilisation de dérivés de l'adénosine selon la revendication 1 caractérisés en ce que R est un groupe méthyle, éthyle, propyle , isopropyle, butyle, isobutyle, sec-butyle, pentyle, hexyle, heptyle, octyle, décyle, hexadécyle, octadécyle, benzyle; $R_1$ est un groupe hydrogène, acétyle, propionyle, butyryle, succinyle, glutaryle, benzoyle, tosyle; et $R_2$ est un groupe hydrogène, acétyle, propionyle, butyryle, succinyle, glutaryle, benzoyle, tosyle.

3. Utilisation de dérivés de l'adénosine selon la revendication 1 caractérisés en ce que $R_1$=$R_2$=H, R=$CH_3$.

4. Utilisation de dérivés de l'adénosine selon la revendication 1 caractérisés par l'addition d'un sel acide pharmacologiquement acceptable quand $R_1$=H.

5. Utilisation de dérivés de l'adénosine selon la revendication 4 caractérisés en ce que le sel acide ajouté est un chlorure, un sulfate, un sulfate d'hydrogène, un phosphate, un formate, un acétate, un citrate, un tartrate, un lactate, un méthanesulfonate, un p-toluènesulfonate.

6. Utilisation de dérivés de l'adénosine selon la revendication 1, caractérisés par le fait que lesdits composés sont utilisables pour une administration orale, parentérale, intraveineuse, rectale ou topique.

7. Utilisation de dérivés de l'adénosine selon la revendication 1 caractérisés par le fait que lesdits composés sont préparés sous forme de gélules contenant de 50 à 100 mg du dérivé de formule (I) dans laquelle $R_1$=$R_2$=H et R=$CH_3$.

8. Utilisation de dérivés de l'adénosine selon la revendication 1, caractérisés en ce que lesdits composés sont préparés sous forme de comprimés contenant de 50 à 100 mg du dérivé de formule (I) dans laquelle $R_1$=$R_2$=H et R=$CH_3$.

9. Utilisation de dérivés de l'adénosine selon la revendication 1 caractérisés en ce que lesdits composés sont préparés sous forme de suppositoires contenant de 50 à 100 mg du dérivé de formule (I) dans laquelle $R_1$=$R_2$=H et R=$CH_3$.

10. Utilisation de dérivés de l'adénosine selon la revendication 1 caractérisés en ce que lesdits composés sont préparés sous forme d'ampoules injectables contenant de 25 à 50 mg du dérivé de formule (I) dans laquelle $R_1$=$R_2$=H et R=$CH_3$.

11. Utilisation de dérivés de l'adénosine selon la revendication (I) caractérisés en ce que lesdits composés sont préparés sous forme d'ampoules buvables contenant de 50 à 100 mg du dérivé de formule (I) dans laquelle $R_1$=$R_2$=H et R=$CH_3$.

**Revendications pour les Etats contractants suivants : GR, ES**

1. Procédé de préparation de composés pharmaceutiques immunostimulants indiqués dans le traitement thérapeutique d'immunodéficiences congénitales ou acquises et de tumeurs, contenant comme principe actif un dérivé de l'adénosine de formule générale (I) :

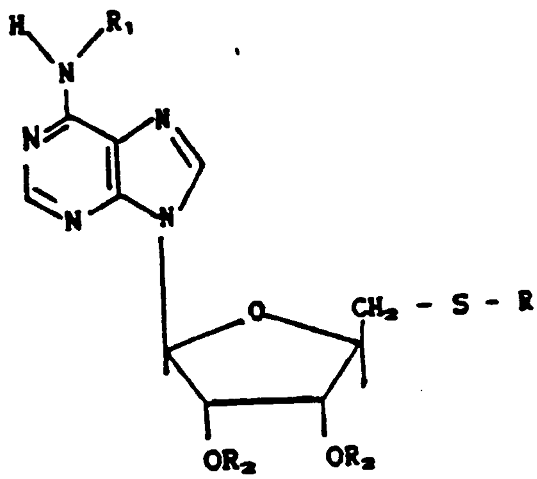

(I)

dans laquelle :

R = $C_1$-$C_{18}$ est un radical alkyle linéaire ou ramifié ou un radical phénylalkylène dans lequel la chaîne alkylène a de 1 à 6 atomes de carbone;

$R_1$ = H, $C_1$-$C_6$ est un radical acyle aliphatique ou un radical acyle aromatique $C_7$;

$R_2$ = H, $C_1$-$C_6$ est un radical acyle aliphatique, ou $C_7$ un radical acyle aromatique, ou les deux radicaux $R_2$ forment ensemble une chaîne isopropylidène, en combinaison avec des excipients pharmaceutiques usuels compatibles et le dérivé de l'adénosine.

2. Procédé selon la revendication 1 caractérisé en ce que R est un groupe méthyle, éthyle, propyle , isopropyle, butyle, isobutyle, sec-butyle, pentyle, hexyle, heptyle, octyle, décyle, hexadécyle, octadécyle, benzyle; $R_1$ est un groupe hydrogène, acétyle, propionyle, butyryle, succinyle, glutaryle, benzoyle, tosyle; et $R_2$ est un groupe hydrogène, acétyle, propionyle, butyryle, succinyle, glutaryle, benzoyle, tosyle.

3. Procédé selon la revendication 1 caractérisé en ce que $R_1$=$R_2$=H, R=$CH_3$.

4. Procédé selon la revendication 1 caractérisé par l'addition d'un sel acide pharmacologiquement acceptable lorsque $R_1$=H.

5. Procédé selon la revendication 4, caractérisé en ce que le sel acide ajouté est un chlorure, un sulfate, un sulfate d'hydrogène, un phosphate, un formate, un acétate, un citrate, un tartrate, un lactate, un méthanesulfonate, un p-toluènesulfonate.

6. Procédé selon la revendication 1 caractérisé en ce que lesdits composés sont utilisables pour une administration orale, parentérale, intraveineuse, rectale ou topique.

7. Procédé selon la revendication 1 caractérisé en ce que lesdits composés sont préparés sous forme de gélules contenant de 50 à 100 mg du dérivé de formule (I) dans laquelle $R_1$=$R_2$=H et R=$CH_3$.

8. Procédé selon la revendication 1 caractérisé en ce que lesdits composés sont préparés sous forme de comprimés contenant de 50 à 100 mg du dérivé de formule (I) dans laquelle $R_1$=$R_2$=H et R=$CH_3$.

9. Procédé selon la revendication 1 caractérisé en ce que lesdits composés sont préparés sous forme de suppositoires contenant de 50 à 100 mg du dérivé de formule (I) dans laquelle $R_1$=$R_2$=H et R=$CH_3$.

10. Procédé selon la revendication 1 caractérisé en ce que lesdits composés sont préparés sous forme d'ampoules injectables contenant de 25 à 50 mg du dérivé de formule (I) dans laquelle $R_1$=$R_2$=H et R=$CH_3$.

11. Procédé selon la revendication 1 caractérisé en ce que lesdits composés sont préparés sous forme d'ampoules buvables contenant de 50 à 100 mg du dérivé de formule (I) dans laquelle $R_1$=$R_2$=H et R=$CH_3$.